# EUROPEAN PATENT APPLICATION

(11) **EP 4 382 142 A1**
(43) Date of publication of application: **12.06.2024**
(21) Application number: 22853342.8
(22) Date of filing: 27.07.2022
(51) Int. Cl.: A61L 27/52, A61L 27/46, A61L 27/56, A61L 27/12, A61L 27/54, A61L 27/38

(54) **COMPOSITION FOR PREPARING ORGANIC-INORGANIC COMPLEX HYDROGEL AND KIT FOR PREPARING ORGANIC-INORGANIC COMPLEX HYDROGEL COMPRISING SAME**

(30) Priority: 06.08.2021 KR 20210103711; 26.07.2022 KR 20220092331
(71) Applicant: KOREA INSTITUTE OF MATERIALS SCIENCE, Gyeongsangnam-do 51508 (KR)
(72) Inventor: PARK, Hong-hyun, Changwon-si Gyeongsangnam-do 51448 (KR); YUN, Hui-suk, Changwon-si Gyeongsangnam-do 51667 (KR); CHOI, Yeong-jin, Changwon-si Gyeongsangnam-do 51511 (KR)
(74) Representative: Vossius & Partner Patentanwälte Rechtsanwälte mbB
(86) International application number: PCT/KR2022/011063
(87) International publication number: WO 2023/013964

(57) **Abstract**

The present invention relates to a composition for preparing an organic-inorganic complex hydrogel and a kit for preparing an organic-inorganic complex hydrogel comprising same, and specifically, to a composition for preparing an organic-inorganic complex hydrogel and a kit for preparing an organic-inorganic complex hydrogel comprising same, comprising: a biocompatible polymer having a photocrosslinkable functional group; and a calcium phosphate-based ceramic powder.

## Description

### BACKGROUND OF THE INVENTION

### 1. Field of the Invention

The present invention relates to a composition for preparing an organic-inorganic complex hydrogel and a kit for preparing an organic-inorganic complex hydrogel comprising the same.

### 2. Description of the Related Art

Tissue engineering has been known as a method to regenerate or replace human biological tissues or organs damaged by disease or accident. According to this method, artificial tissues and organs can be manufactured, typically with the use of the patient's own cells and polymer supports.

Briefly, after collecting a part of a biological tissue from a patient, only the necessary cells are separated and cultured to secure a sufficient number of cells. The cells can be grown into a biological tissue in a porous polymer support with a three-dimensional structure and then be transplanted back to the patient through surgery. Or, the cells can be mixed with a support such as a hydrogel and then injected into a living body using a syringe to regenerate biological tissues. The polymer support used at this time plays various roles of the extracellular matrix (ECM) of biological tissues. The polymer support participates in the attachment, proliferation, and differentiation of cells and controls the function and structure of the biological tissue to be regenerated. The polymer support also regulates the diffusion of water-soluble factors, nutrients, and metabolites. In particular, the interaction between polymer supports and cells is a very important factor in tissue engineering. Various studies are being conducted on biological interactions, physical properties of polymer supports, and control of the release of soluble factors from supports to regulate the interaction between cells and supports. By appropriately adjusting these factors to control the growth and differentiation of cells, a desired biological tissue can be successfully regenerated.

As such a polymer support, a hydrogel with a three-dimensional hydrophilic polymer network structure capable of containing a large amount of water has recently received the most attention due to the high water content and structural properties similar to the extracellular matrix (ECM) structure. However, due to the low mechanical strength of the hydrogel, research has been conducted to improve its properties.

As a related prior art, Patent Document 1 proposed a cell-laden biocompatible polymer-biocompatible natural material hybrid scaffold producing method consisting of the following steps: (a) a step of forming a strut layer by distributing two or more biocompatible polymer struts side by side on a plate; (b) a step of distributing biocompatible polymer struts side by side at intervals in a direction crossing the direction of the distributed biocompatible polymer struts on the distributed biocompatible polymer strut layer; (c) a step of forming a cross-link in the biocompatible natural material being distributed while distributing a strut comprising at least one natural biocompatible material selected from the group consisting of cell-laden gelatin, fucoidan, collagen, alginate, chitosan and hyaluronic acid between the biocompatible polymer struts distributed in step (b), but not in contact with the biocompatible polymer struts; and (d) a step of forming a hybrid structure by sequentially repeating steps (b) and (c) above.

In addition, Patent Document 2 proposed a polymer-ceramic hybrid film in which flexibility and mechanical properties are controlled by adjusting the mixing ratio of polymer and ceramic within a specific range, and a method for preparation thereof.

However, the method proposed in Patent Document 1 (Korean Patent No. 10-1360942) has a disadvantage that it requires a heat treatment process, which makes it difficult to manufacture at room temperature and cannot be manufactured in various shapes. The method proposed in Patent Document 2 (Korean Patent Publication No. 10-2019-0057268) was able to improve the mechanical properties of the hydrogel to some extent, but there is a problem in that the hydrogel is difficult to form when more ceramics are used to improve the mechanical strength.

To solve the above problems, the present inventors have developed a composition for preparing an organic-inorganic complex hydrogel comprising a photopolymerizable biocompatible polymer and a calcium phosphate ceramic powder that can be cured at room temperature by a hydration reaction as a composition for preparing an organic-inorganic complex hydrogel that has a high water content and exhibits remarkably excellent mechanical properties, and a kit containing the composition and a curing liquid, resulting in the completion of the present invention.

### SUMMARY OF THE INVENTION

An object of the present invention in one aspect is to provide an organic-inorganic complex hydrogel composition for forming a support for tissue regeneration and a kit comprising the same.

To achieve the above object, in one aspect of the present invention, the present invention provides a composition for preparing an organic-inorganic complex hydrogel comprising a biocompatible polymer having a photocrosslinkable functional group; and a calcium phosphate-based ceramic powder.

The weight ratio of the biocompatible polymer and the calcium phosphate-based ceramic powder may be1:1 or more and less than 1:20, preferably1:2 or more and less than 1:20, more preferably 1:5 to 1:15, and most preferably 1:7 to 1:12.

The biocompatible polymer may be at least one selected from the group consisting of alginate, hydroxypropylmethylcellulose (HPMC), hydroxyethylcellulose (HEC), methyl cellulose, carboxymethylcellulose (CMC), gelatin, collagen, fibrinogen, chitosan, agar, matrigel, starch, pectin, polyvinyl alcohol, polyurethane, poly(ethylene glycol), poly(propylene glycol), hyaluronan and poly(vinylpyrrolidone).

The calcium phosphate-based ceramic powder may be at least one selected from the group consisting of TCP (tricalcium phosphate), hydroxyapatite, DCPD (dicalcium phosphate dihydrate, MCPM (monocalcium phosphate monohydrate), DCPA (dicalcium phosphate anhydrous) and BCP (biphasic calcium phosphate), and preferably may be α-TCP (a-tricalcium phosphate).

The composition for preparing an organic-inorganic complex hydrogel may further include at least one of a functional member and a cell.

In another aspect of the present invention, the present invention provides a kit for preparing an organic-inorganic complex hydrogel comprising a composition for preparing an organic-inorganic complex hydrogel comprising a biocompatible polymer having a photocrosslinkable functional group and a calcium phosphate-based ceramic powder; and a curing liquid.

The weight ratio of the biocompatible polymer and the calcium phosphate-based ceramic powder may be1:1 or more and less than 1:20, preferably1:2 or more and less than 1:20, more preferably 1:5 to 1:15, and most preferably 1:7 to 1:12.

The curing liquid may be at least one selected from the group consisting of saline, PBS (phosphate buffer saline), MCPM (monocalcium phosphate monohydrate), DSP (disodium phosphate dehydrate), MSP (monosodium phosphate dehydrate), a-MEM (minimum essential medium) and HBSS (Hank's balanced salt solution).

The curing liquid can further include calcium ions (Ca²⁺).

The kit may further include at least one of a functional member and a cell.

In another aspect of the present invention, the present invention provides an organic-inorganic complex hydrogel comprising a biocompatible polymer having a photocrosslinkable functional group; and a calcium phosphate-based ceramic bonded to the biocompatible polymer.

The calcium phosphate-based ceramic is CDHA (Ca-deficient hydroxyapatite).

In another aspect of the present invention, the present invention provides a biomaterial comprising the organic-inorganic complex hydrogel.

### ADVANTAGEOUS EFFECT

According to one aspect, the composition for preparing an organic-inorganic complex hydrogel can be used to produce an organic-inorganic complex hydrogel with high water content and excellent mechanical properties at room temperature.

According to one aspect, the composition for preparing an organic-inorganic complex hydrogel can be used to produce an organic-inorganic complex hydrogel with remarkably excellent mechanical properties by photocrosslinking a biocompatible polymer through UV irradiation and curing a calcium phosphate-based ceramic through a curing liquid.

According to another aspect, the kit for preparing an organic-inorganic complex hydrogel is a kit comprising a composition for preparing an organic-inorganic complex hydrogel and a curing liquid, and the composition has high fluidity, so it is easy to inject into the human body and has an excellent cell adhesion effect after curing at room temperature. Thus, the kit can deliver drugs or cells to a desired location in the body through minimal surgical intervention by curing using UV irradiation and a curing liquid after being injected into the body, and the prepared organic-inorganic complex hydrogel can be used as a support for tissue regeneration.

The effects of the present invention are not limited to those described above, and should be understood to include all effects that can be inferred from the detailed description of the invention or from the configuration of the invention described in the claims.

### BRIEF DESCRIPTION OF THE DRAWINGS

Figures 1 to 5 are graphs showing the results of measuring the size of the hydrogels according to Examples and Comparative Examples prepared by varying the weight ratio of the biocompatible polymer and the calcium phosphate ceramic powder before and after curing by a curing liquid.
Figures 6 to 11 are graphs comparing the compressive strength values for each of the hydrogels according to Examples and Comparative Examples prepared by varying the weight ratio of the biocompatible polymer and the calcium phosphate ceramic powder and the type of a curing liquid.
Figure 12 is a graph showing the results of measuring the crystal structure of the hydrogels according to Examples and Comparative Examples prepared by varying the weight ratio of the biocompatible polymer and the calcium phosphate ceramic powder using an X-ray diffraction (XRD) device before curing by a curing liquid.
Figure 13 is a graph showing the results of measuring the crystal structure of the hydrogels according to Examples and Comparative Examples prepared by varying the weight ratio of the biocompatible polymer and the calcium phosphate ceramic powder using an X-ray diffraction (XRD) device after curing by a curing liquid.
Figure 14 is a graph showing the results of measuring the water content of the hydrogels according to Examples and Comparative Examples prepared by varying the weight ratio of the biocompatible polymer and the calcium phosphate ceramic powder before freeze-drying.
Figure 15 is a graph showing the results of measuring the water content of the hydrogels according to Examples and Comparative Examples prepared by varying the weight ratio of the biocompatible polymer and the calcium phosphate ceramic powder after freeze-drying.
Figure 16 is a photograph showing the surfaces of the hydrogels according to Examples and Comparative Examples prepared by varying the weight ratio of the biocompatible polymer and the calcium phosphate ceramic powder, observed under a scanning electron microscope (SEM).
Figure 17 is a photograph showing the results of observing the cell adhesion capacity under a fluorescence microscope after culturing the octadecyl rhodamine B-stained cells on the hydrogels according to Examples and Comparative Examples prepared by varying the weight ratio of the biocompatible polymer and the calcium phosphate ceramic powder.
Figure 18 is a photograph showing the state after photocrosslinking and ionic crosslinking of the compositions according to Examples and Comparative Examples observed to determine whether or not a hydrogel is formed.
Figure 19 is a photograph showing the state after photocrosslinking of the composition in which the biocompatible polymer and the calcium phosphate ceramic powder are mixed in a weight ratio of 1:20 according to an embodiment observed to determine whether or not a hydrogel is formed.
Figure 20 is a graph showing the initial drug loading efficiency of the hydrogels according to Examples and Comparative Examples prepared by varying the weight ratio of the biocompatible polymer and the calcium phosphate ceramic powder.
Figure 21 is a graph showing the release behavior of the hydrogels according to Examples and Comparative Examples prepared by varying the weight ratio of the biocompatible polymer and the calcium phosphate ceramic powder for one month after drug loading.
Figure 22 is a graph showing the results of measuring the DNA content of the cells on the surface after culturing MG-63 cells on the surface of the hydrogel for 2 weeks in order to confirm the possibility of inducing osteogenic differentiation of the hydrogels according to Examples and Comparative Examples prepared by varying the weight ratio of the biocompatible polymer and the calcium phosphate ceramic powder.
Figure 23 is a graph showing the results of measuring the ALP activity of the cells on the surface after culturing MG-63 cells on the surface of the hydrogel for 2 weeks in order to confirm the possibility of inducing osteogenic differentiation of the hydrogels according to Examples and Comparative Examples prepared by varying the weight ratio of the biocompatible polymer and the calcium phosphate ceramic powder.
Figure 24 is a photograph showing the experimental process for confirming curing by UV after injection of the compositions according to Examples and Comparative Examples into the living tissue.
Figure 25 is a photograph showing the experimental results of Figure 12.
Figure 26 is a graph showing the compressive strength values for the hydrogels according to Examples and Comparative Examples cured by UV after injection into the living tissue.

### DESCRIPTION OF THE PREFERRED EMBODIMENTS

Hereinafter, the preferable embodiments of the present invention are described with the attached drawings. However, the embodiments of the present invention can be modified and altered in various ways and the present invention is not limited to the following illustration. It is well understood by those in the art who has the average knowledge on this field that the embodiments of the present invention are given to explain the present invention more precisely. Therefore, the shape and size of the elements in the drawings may be exaggerated for clarity of illustration and the elements indicated by the same mark in the drawings are the same elements. The factors showing similar function or activity are also indicated by the same mark in all the drawings. In addition, the "inclusion" of an element throughout the specification does not exclude other elements, but may include other elements, unless specifically stated otherwise.

In one aspect of the present invention, the present invention provides a composition for preparing an organic-inorganic complex hydrogel comprising a biocompatible polymer having a photocrosslinkable functional group; and a calcium phosphate-based ceramic powder.

The composition for preparing an organic-inorganic complex hydrogel according to one aspect can be used to prepare an organic-inorganic complex hydrogel with high water content and excellent mechanical properties.

In addition, the composition for preparing an organic-inorganic complex hydrogel according to one aspect can be used to prepare an organic-inorganic complex hydrogel with excellent cell adherence.

Accordingly, the composition for preparing an organic-inorganic complex hydrogel according to one aspect may be a composition for preparing a hydrogel for use as a biomaterial such as a support for tissue regeneration, but not always limited thereto.

Hereinafter, the composition for preparing an organic-inorganic complex hydrogel according to one aspect will be described in detail.

The composition for preparing an organic-inorganic complex hydrogel according to one aspect includes a biocompatible polymer having a photocrosslinkable functional group.

The biocompatible polymer may be at least one selected from the group consisting of alginate, hydroxypropylmethylcellulose (HPMC), hydroxyethylcellulose (HEC), methyl cellulose, carboxymethylcellulose (CMC), gelatin, collagen, fibrinogen, chitosan, agar, matrigel, starch, pectin, polyvinyl alcohol, polyurethane, poly(ethylene glycol), poly(propylene glycol), hyaluronan and poly(vinylpyrrolidone).

The molecular weight of the biocompatible polymer may be 5,000 Da to 5,000,000 Da, and more preferably 10,000 Da to 1,000,000 Da.

The biocompatible polymer has a photocrosslinkable functional group, through which it can be crosslinked by light.

At this time, the photocrosslinkable functional group may be at least one selected from the group consisting of a methacrylate group, an acrylate group, a vinyl group, an epoxy group, and a tyrosine group.

For example, the biocompatible polymer can be an alginate having a methylate group.

If the biocompatible polymer does not include a photocrosslinkable functional group, the preparation of an organic-inorganic complex hydrogel using the above composition may result in problems such as failure to produce hydrogels due to poor crosslinking or preparation of hydrogels with lower mechanical strength.

The composition for preparing an organic-inorganic complex hydrogel according to one aspect includes a calcium phosphate-based ceramic powder.

The calcium phosphate-based ceramic powder may be at least one selected from the group consisting of α-TCP (a-tricalcium phosphate), β-TCP (β-tricalcium phosphate), hydroxyapatite, DCPD (dicalcium phosphate dihydrate), MCPM (monocalcium phosphate monohydrate), DCPA (dicalcium phosphate anhydrous) and BCP (biphasic calcium phosphate). However, it may be more preferred that the calcium phosphate-based ceramic powder is α-TCP, which can be cured by a hydration reaction at room temperature.

Specifically, the α-TCP can be cured while being phase transformed into CDHA (Ca-deficient hydroxyapatite) having excellent cell adhesion ability and mechanical strength through a hydration reaction with a curing liquid such as PBS at room temperature.

The composition for preparing an organic-inorganic complex hydrogel according to one aspect can form an organic-inorganic complex hydrogel at room temperature by light irradiation and immersion in a curing liquid. The formed hydrogel can have remarkably excellent mechanical properties because it is bonded by photocrosslinking of the hydrogel and curing of the ceramic powder.

The composition for preparing an organic-inorganic complex hydrogel according to one aspect includes an amount of the calcium phosphate-based ceramic powder equal to or greater than the weight of the biocompatible polymer.

The composition may include the biocompatible polymer and the calcium phosphate-based ceramic powder in a weight ratio of 1:1 or more and less than 1:20, preferably1:2 or more and less than 1:20, more preferably 1:5 to 1:15, and most preferably 1:7 to 1:12.

If the weight of the calcium phosphate-based ceramic powder is less than the weight of the biocompatible polymer, the mechanical strength of the organic-inorganic complex hydrogel produced may be low, making it difficult to use for purposes requiring higher mechanical strength, and it may also be difficult to use as a biomaterial such as a support for tissue regeneration due to its low cell adhesion ability.

In addition, if the weight of the calcium phosphate-based ceramic powder is 20 times or more than the weight of the biocompatible polymer, the photocrosslinking of the polymer may not be properly accomplished, and thus an organic-inorganic complex hydrogel may not be formed.

The composition for preparing an organic-inorganic complex hydrogel according to one aspect may further include at least one of a functional member and a cell.

The functional member may include a drug, a protein such as a growth factor, and the like.

The composition for preparing an organic-inorganic complex hydrogel according to one aspect may further include at least one of a functional member and a cell to be used for the purpose of delivering cells, growth factors, drugs, and the like in vivo, and may also be used as a biomaterial such as a support for tissue regeneration in vivo.

In another aspect of the present invention, the present invention provides a kit for preparing an organic-inorganic complex hydrogel comprising a composition for preparing an organic-inorganic complex hydrogel comprising a biocompatible polymer having a photocrosslinkable functional group and a calcium phosphate-based ceramic powder; and a curing liquid.

Hereinafter, the kit for preparing an organic-inorganic complex hydrogel according to another aspect will be described in detail.

The kit for preparing an organic-inorganic complex hydrogel may include a composition for preparing an organic-inorganic complex hydrogel and a curing liquid for curing the calcium phosphate-based ceramic powder included in the composition at room temperature.

The composition for preparing an organic-inorganic complex hydrogel may include a configuration of the composition for preparing an organic-inorganic complex hydrogel described above.

An aqueous solution may be used as the curing liquid, but a culture medium used for cell culture may be preferably used to increase the biocompatibility of the organic-inorganic hydrogel to be prepared.

The curing liquid may be at least one selected from the group consisting of saline, PBS (phosphate buffer saline), MCPM (monocalcium phosphate monohydrate), DSP (disodium phosphate dehydrate), MSP (monosodium phosphate dehydrate), a-MEM (minimum essential medium) and HBSS (Hank's balanced salt solution).

The kit for preparing an organic-inorganic complex hydrogel is a kit for preparing a hydrogel for use as a biomaterial such as a support for tissue regeneration, and may further include at least one of a functional member and a cell. The functional member may include a drug, a protein such as a growth factor, and the like.

An organic-inorganic complex hydrogel can be prepared by the following method using the kit for preparing an organic-inorganic complex hydrogel.

Specifically, the preparation process includes the following steps: a step of photocrosslinking the composition for preparing an organic-inorganic complex hydrogel comprising a biocompatible polymer having a photocrosslinkable functional group and a calcium phosphate-based ceramic powder by irradiating light; and a step of immersing the photocrosslinked composition in a curing liquid

First, a step of photocrosslinking the composition for preparing an organic-inorganic complex hydrogel by irradiating light such as UV is performed.

The step is a step of photocrosslinking the biocompatible polymer included in the composition to form a hydrogel.

The composition may form a hydrogel having excellent mechanical strength by light irradiation.

For example, when a solution containing alginate and α-TCP in a weight ratio of 1:10 is irradiated with UV, crosslinking of the alginate is well formed, whereas when ion crosslinking is performed by immersion in a solution containing calcium ions, crosslinking of the alginate may not be properly formed.

Since the composition for preparing an organic-inorganic complex hydrogel has fluidity, it can be easily delivered to a target position in the body in a minimum invasive method. Thus, the step of photocrosslinking the composition by irradiating light such as UV can be performed after delivering the composition to a target position in the body.

The composition may include the biocompatible polymer and the calcium phosphate-based ceramic powder in a weight ratio of 1:1 or more and less than 1:20, preferably1:2 or more and less than 1:20, more preferably 1:5 to 1:15, and most preferably 1:7 to 1:12.

By including the biocompatible polymer and the calcium phosphate-based ceramic powder in the above weight ratio, the composition for preparing an organic-inorganic complex hydrogel can be used to prepare a hydrogel having high water content and significantly excellent mechanical strength.

Next, a step of immersing the photocrosslinked composition in a curing liquid is performed.

The step is a step of curing the ceramic powder contained in the hydrogel formed by photocrosslinking.

At this time, an aqueous solution may be used as the curing liquid, but a culture medium used for cell culture may be preferably used to increase the biocompatibility of the organic-inorganic hydrogel to be prepared.

The hydrogel containing the ceramic powder formed by UV irradiation after injection into the body can be cured by injecting the culture medium or can be cured by body fluid without injecting a separate curing liquid.

The curing liquid may be preferably PBS (phosphate buffer saline) in order to further improve the mechanical strength of the organic-inorganic hydrogel to be prepared, and more preferably PBS (phosphate buffer saline) including calcium ions (Ca²⁺).

In another aspect of the present invention, the present invention provides an organic-inorganic complex hydrogel comprising a biocompatible polymer having a photocrosslinkable functional group; and a calcium phosphate-based ceramic bonded to the biocompatible polymer.

The organic-inorganic complex hydrogel includes a biocompatible polymer having a photocrosslinkable functional group and a calcium phosphate-based ceramic bonded to the biocompatible polymer, and the calcium phosphate-based ceramic may preferably be CDHA (Ca-deficient hydroxyapatite).

The organic-inorganic complex hydrogel has a high water content and excellent mechanical strength and cell adhesion ability, so it can be used as a biomaterial such as a support for tissue regeneration.

In another aspect of the present invention, the present invention provides a biomaterial comprising the organic-inorganic complex hydrogel.

Hereinafter, the present invention will be described in detail by the following examples and experimental examples.

However, the following examples and experimental examples are only for illustrating the present invention, and the contents of the present invention are not limited thereto.

### <Preparative Example 1>

3 g of sodium alginate (molecular weight: 200,000-300,000; FMC Biopolymer) was dissolved in 300 ml of 0.1 M 2-(N-morpholino)ethanesulfonic acid (MES) buffer (0.3 M NaCl, pH = 6.5), 1.628 g of N-hydroxysuccinimide (sulfo-NHS), 2.875 g of ethylene dichloride (EDC), and 1.234 g of N-(2-aminoethyl)methacrylamide hydrochloride (AEMA) were mixed therein, followed by synthesis for 20 hours. After synthesis, it was dialyzed using distilled water for 4 days, treated with activated carbon, filtered through a 0.22 µm filter, and lyophilized to prepare an alginate having a methacrylate group.

### <Example 1> Composition for preparing organic-inorganic complex hydrogel

An alginate solution was prepared by dissolving 0.087 g of the alginate prepared in Preparative Example 1 in distilled water. Then, 0.0435 g of α-TCP (a-tricalcium phosphate) was added to the solution and dispersed using a three-dimensional ultrasonic mixer to prepare a composition for preparing an organic-inorganic complex hydrogel with a weight ratio of the alginate and α-TCP of 1:0.5.

### <Example 2> Composition for preparing organic-inorganic complex hydrogel

A composition for preparing an organic-inorganic complex hydrogel with a weight ratio of the alginate and α-TCP of 1:1 was prepared in the same manner as in Example 1, except that the weight of α-TCP in Example 1 was changed to 0.087 g.

### <Example 3> Composition for preparing organic-inorganic complex hydrogel

A composition for preparing an organic-inorganic complex hydrogel with a weight ratio of the alginate and α-TCP of 1:2 was prepared in the same manner as in Example 1, except that the weight of α-TCP in Example 1 was changed to 0.174 g.

### <Example 4> Composition for preparing organic-inorganic complex hydrogel

A composition for preparing an organic-inorganic complex hydrogel with a weight ratio of the alginate and α-TCP of 1:5 was prepared in the same manner as in Example 1, except that the weight of α-TCP in Example 1 was changed to 0.435 g.

### <Example 5> Composition for preparing organic-inorganic complex hydrogel

A composition for preparing an organic-inorganic complex hydrogel with a weight ratio of the alginate and α-TCP of 1:10 was prepared in the same manner as in Example 1, except that the weight of α-TCP in Example 1 was changed to 0.87 g.

### <Example 6> Composition for preparing organic-inorganic complex hydrogel

A composition for preparing an organic-inorganic complex hydrogel with a weight ratio of the alginate and α-TCP of 1:20 was prepared in the same manner as in Example 1, except that the weight of α-TCP in Example 1 was changed to 1.74 g.

### <Example 7> Organic-inorganic complex hydrogel

The solutions of Examples 1 to 6 were photocrosslinked by UV irradiation, and then immersed in each of the curing liquids below at 37°C for about 25 hours to prepare organic-inorganic complex hydrogels.

At this time, α-MEM (minimum essential medium), PBS (phosphate buffer saline), PBS (phosphate buffer saline) containing about 0.1 g/L of calcium chloride (CaCl₂), HBSS (Hank's balanced salt solution), and saline were used as the curing liquid.

### <Comparative Example 1>

An alginate solution was prepared by dissolving 0.087 g of the alginate prepared in Preparative Example 1 in 5 ml of distilled water.

### <Comparative Example 2>

The solution of Comparative Example 1 was photocrosslinked by UV irradiation, and then immersed in each of the curing liquids below at 37°C for about 25 hours to prepare a hydrogel.

At this time, α-MEM (minimum essential medium), PBS (phosphate buffer saline), PBS (phosphate buffer saline) containing about 0.1 g/L of calcium chloride (CaCl₂), HBSS (Hank's balanced salt solution), and saline were used as the curing liquid.

### <Comparative Example 3>

The solutions of Comparative Example 1 (α-TCP/alginate=0), Example 2 (α-TCP/alginate=1), Example 5 (α-TCP/alginate=10), and Example 6 (α-TCP/alginate=20) above were ionic-crosslinked by adding calcium ions (Ca²⁺), and then immersed in each of the following PBS (phosphate buffer saline) curing liquids at 37°C for about 25 hours to prepare organic-inorganic complex hydrogels.

### <Experimental Example 1> Size change analysis

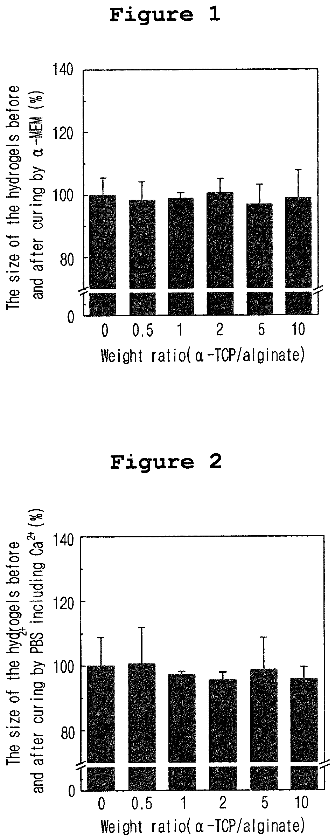

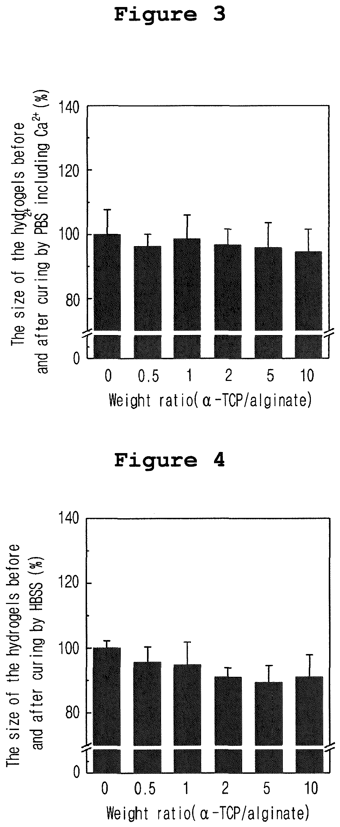

In the process of preparing the organic-inorganic hydrogel according to one aspect, the size of the hydrogel before and after curing by the curing liquid was measured using an ImageJ program by varying the weight ratio of the biocompatible polymer and the calcium phosphate-based ceramic powder and the type of the curing liquid in Example 7 and Comparative Example 2. The results are shown in Figures 1 to 5.

The horizontal axes of Figures 1 to 5 represent the weight ratio of α-TCP to the alginate contained in the composition.

As shown in Figures 1 to 5, it can be seen that the size of the hydrogel tended to decrease slightly after curing by the curing liquid compared to after photocrosslinking by UV irradiation and before curing by the curing liquid, but there was no significant difference. Accordingly, when the composition according to one aspect is injected into the body to be used as a tissue substitute material and a regenerative material and then cured in the body, it can be expected to retain its initial volume without volume reduction by the curing liquid.

### <Experimental Example 2> Evaluation of mechanical properties

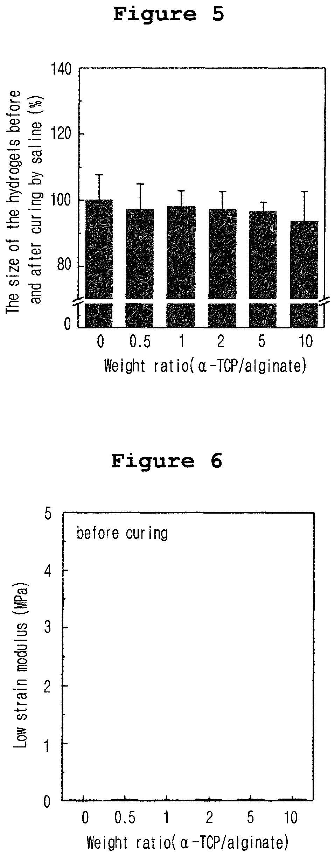

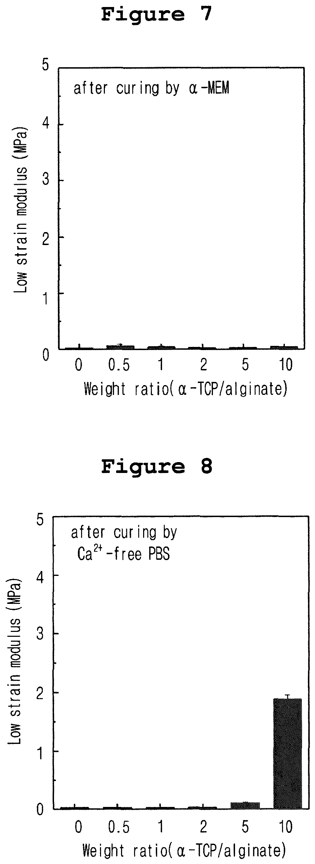

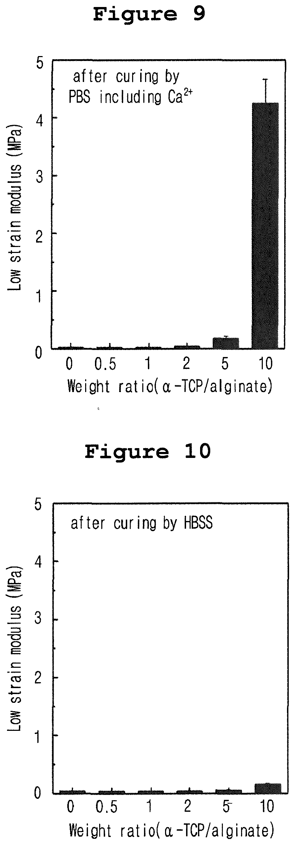

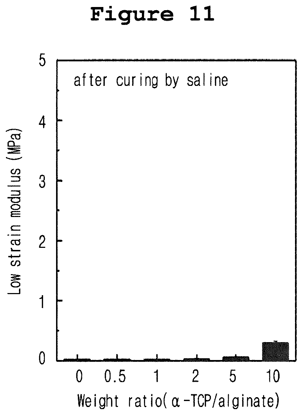

In order to confirm the difference in mechanical properties of the organic-inorganic hydrogel of the present invention according to the use of the curing liquid, the weight ratio of the biocompatible polymer and the calcium phosphate-based ceramic powder, and the type of the curing liquid, the compressive strength of each of the organic-inorganic complex hydrogel prepared in Example 7 and the hydrogel prepared in Comparative Example 2 was measured by the method of axial compression mode using a rotational rheometer (TA instruments). The low modulus values of the stress-strain curves obtained from the above measurements were compared and shown in Figures 6 to 11.

As a result, as shown in Figures 6 to 11, when the curing liquid was not used (before setting), the compression strength did not improve even if the content of α-TCP was increased, whereas when the curing liquid was used, the compression strength was improved as the content of α-TCP was increased.

Through this, it was found that when the calcium phosphate-based ceramic powder was included in the organic-inorganic complex hydrogel, the mechanical properties of the hydrogel were superior to when it was not included. This can be attributed to the fact that curing by the curing liquid of the organic-inorganic complex hydrogel photocrosslinked by UV irradiation did not proceed when α-TCP was not included, whereas curing by the curing liquid proceeded when α-TCP was included.

In addition, regardless of the type of the curing liquid, the hydrogels of Examples 1 to 5 (α-TCP/alginate weight ratio=0.5~10) exhibited higher compressive strength compared to the hydrogel of Comparative Example 1 without α-TCP (α-TCP/alginate weight ratio=0). In particular, the compressive strength was high when the weight ratio of alginate to α-TCP was 5 to 10, and significantly high at 10.

Through this, it was found that when the weight ratio of the biocompatible polymer and the calcium phosphate-based ceramic powder is 5 to 10, especially when the weight ratio is 10, the mechanical properties can be significantly improved.

In addition, in the case of using PBS (PBS w/ Ca) containing calcium ions (Ca²⁺), compared to the case of using other curing liquids and the case of using PBS (PBS w/o Ca) not containing calcium ions, the mechanical properties were significantly improved when the weight ratio of the biocompatible polymer and the calcium phosphate-based ceramic powder was 5 to 10, especially when the weight ratio was 10.

### <Experimental Example 3> Analysis of crystal structure changes

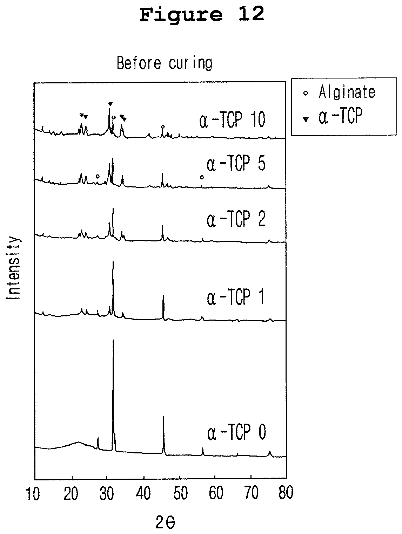

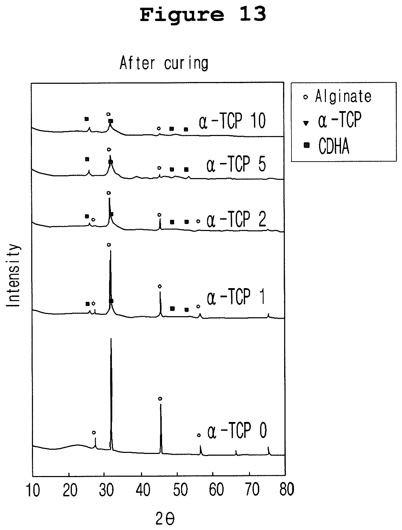

In order to confirm the crystal structure before and after curing by the curing liquid in the process of preparing the organic-inorganic hydrogel according to one aspect, in Example 7 and Comparative Example 2, the crystal structure before and after immersion in the curing liquid was measured using X-ray diffractometry. The results of using PBS as the curing liquid are shown in Figures 12 and 13.

As a result of the crystal structure analysis, alginate and α-TCP, the main components of the composition, existed before immersion in the curing liquid as shown in Figure 12, while after immersion in the curing liquid, alginate and CDHA existed as shown in Figure 13.

This can be attributed to the phase change of α-TCP contained in the composition to CDHA by the curing liquid.

Through the mechanical properties evaluation and crystal structure analysis results, it was found that the mechanical properties of the hydrogel could be significantly improved by mixing the biocompatible polymer and α-TCP, photocrosslinking by UV irradiation, phase-changing the α-TCP to CDHA using the curing liquid to cure, and curing at a low temperature.

### <Experimental Example 4> Evaluation of water content

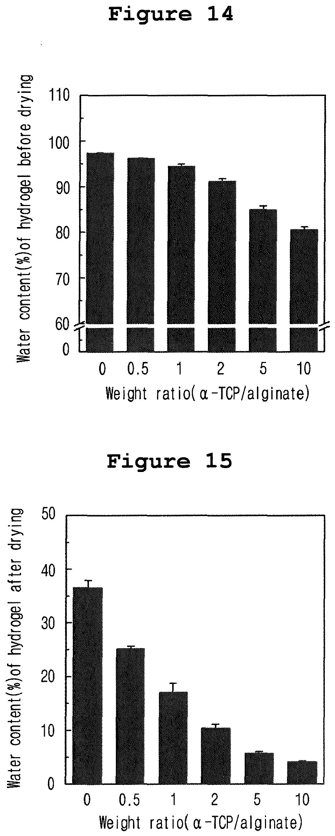

In order to evaluate the water content of the organic-inorganic hydrogel according to one aspect, the water content before (Figure 14) and after (Figure 15) freeze-drying was measured for the organic-inorganic complex hydrogel prepared in Example 7 and the hydrogel prepared in Comparative Example 2. The results of using PBS as the curing liquid are shown in Figures 14 and 15.

As shown in Figures 14 and 15, as a result of comparison before and after freeze-drying, the water content was decreased slightly as the content of α-TCP used was increased, but even when α-TCP was included 10 times the weight of alginate, the water content was higher than 80%. Through this, it was found that the excellent water content of the hydrogel was maintained even when α-TCP was included 10 times the weight of alginate.

In addition, when α-TCP was included 10 times the weight of alginate, it was found that it could contain about 4.2 times more water before freeze-drying compared to after freeze-drying. Through this, it can be expected that it exhibits high cellular affinity and is advantageous for nutrient delivery and gas supply in the body.

### <Experimental Example 5> Surface analysis

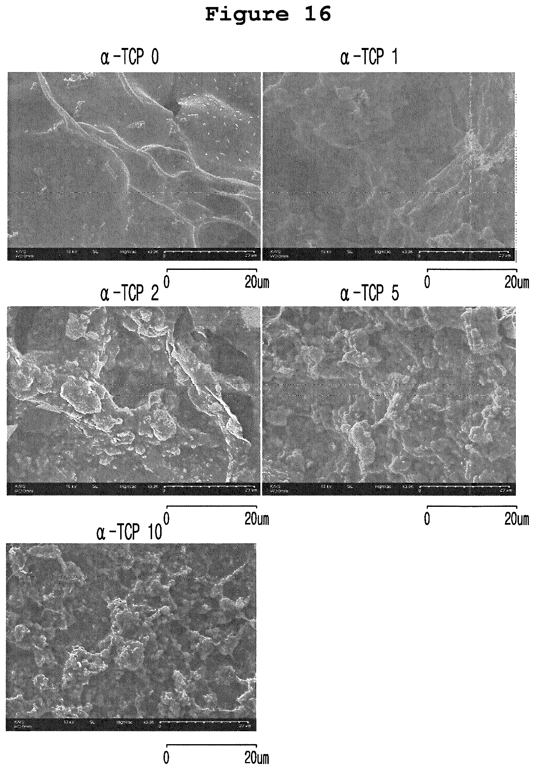

To analyze the surface structure of the organic-inorganic hydrogel according to one aspect, the surface of the organic-inorganic complex hydrogel prepared in Example 7 and the hydrogel prepared in Comparative Example 2 were observed with a scanning electron microscope (SEM). The results of using PBS as the curing liquid are shown in Figure 16.

As shown in Figure 16, as the content of α-TCP was increased, the exposure of α-TCP particles to the surface was increased, and the α-TCP particles were aggregated and connected to each other, and it could be seen that the mechanical properties of the organic-inorganic complex hydrogel were improved through the connection. Accordingly, it was found that a certain percentage of α-TCP should be included in the organic-inorganic complex hydrogel composition to achieve the effect of improving mechanical properties by the ceramic powder.

### <Experimental Example 6> Evaluation of cell adhesion ability

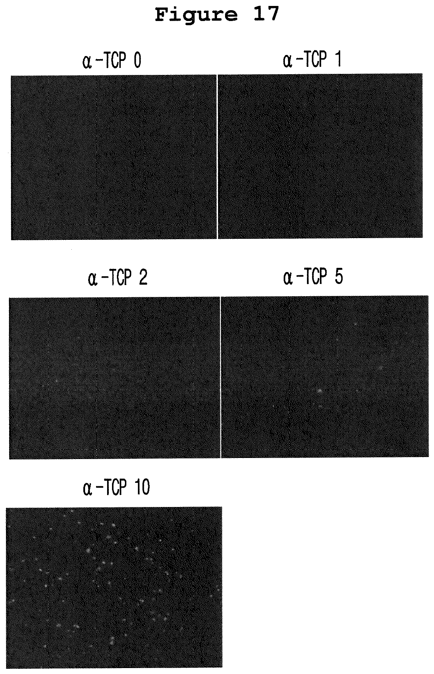

In order to confirm the cell adhesion characteristics of the organic-inorganic hydrogel according to one aspect, cells stained with octadecyl rhodamine B were seeded on the surface of the organic-inorganic complex hydrogel prepared in Example 7 and the hydrogel prepared in Comparative Example 2, and after culture for 24 hours, the degree of cell adhesion was determined by observation under a fluorescence microscope. The results of using PBS as the curing liquid are shown in Figure 17.

As shown in Figure 17, on the hydrogel prepared using the composition of Comparative Example 1 (α-TCP 0) not containing α-TCP, cell culture was hardly performed, whereas on the organic-inorganic complex hydrogel prepared using the composition of Example 2 (α-TCP 1) in which the weight ratio of α-TCP to alginate is 1, cell culture was minimally performed. A larger amount of cells were cultured on the organic-inorganic complex hydrogel prepared using a composition containing more α-TCP, especially a significant number of cells were cultured on the organic-inorganic complex hydrogel prepared using a composition with 5 to 10 times the weight of α-TCP compared to alginate.

This can be attributed to the fact that the CHDA generated by crystallization of α-TCP was exposed on the surface of the organic-inorganic complex hydrogel according to one aspect, and the cell adhesion ability was significantly improved by the CHDA.

### <Experimental Example 7> Differences between photocrosslinking and ionic crosslinking

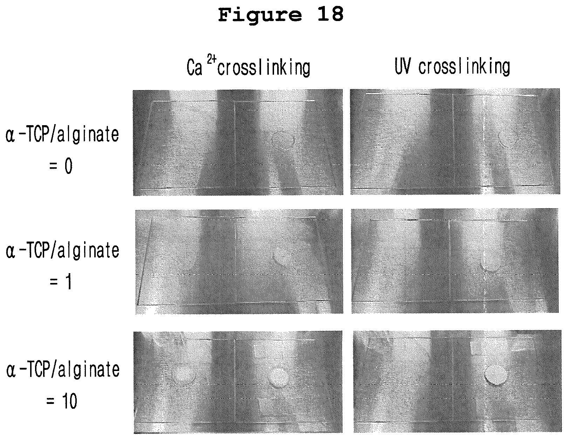

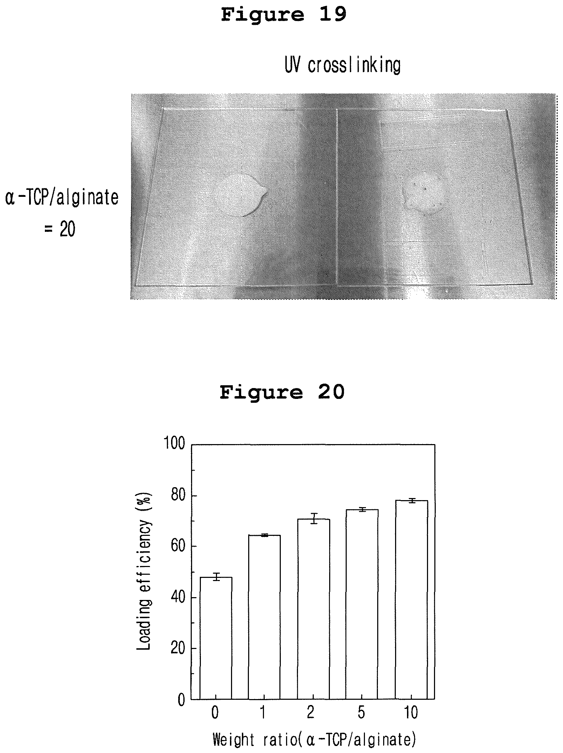

To confirm the differences between photocrosslinking and ionic crosslinking in the formation of organic-inorganic hydrogels, the compositions of Example 2, Example 5, Example 6, and Comparative Example 1 were photocrosslinked by the method of Example 7 to form hydrogels, and the shapes after ionic crosslinking by the method of Comparative Example 3 were compared and observed. The results are shown in Figures 18 and 19.

Figure 18 shows the results of comparative observation of the shapes of the hydrogel formed by photocrosslinking the compositions of Examples 2 and 5, and Comparative Example 1 by the method of Example 7, and ionic crosslinking by the method of Comparative Example 3. As shown in Figure 18, the composition of Comparative Example 1 not containing α-TCP formed hydrogels by both photocrosslinking and ionic crosslinking, whereas the compositions of Examples 2 and 5 containing α-TCP formed hydrogels by photocrosslinking, but did not form hydrogels by ionic crosslinking due to poor crosslinking by calcium ions. This showed that photocrosslinking was more effective than ionic crosslinking to form the composition containing alginate and α-TCP into a hydrogel structure.

Figure 19 shows the results of observing the shape after photocrosslinking the composition of Example 6 by the method of Example 7. As shown in Figure 19, when the weight ratio of alginate and α-TCP was 1:20, it was confirmed that the hydrogel was not formed because the crosslinking was not properly performed even by the photocrosslinking method.

From the above results, it was found that the composition containing alginate and α-TCP can form a hydrogel through photocrosslinking when the weight ratio of α-TCP/alginate is less than 20.

### <Experimental Example 8> Growth factor delivery characteristics

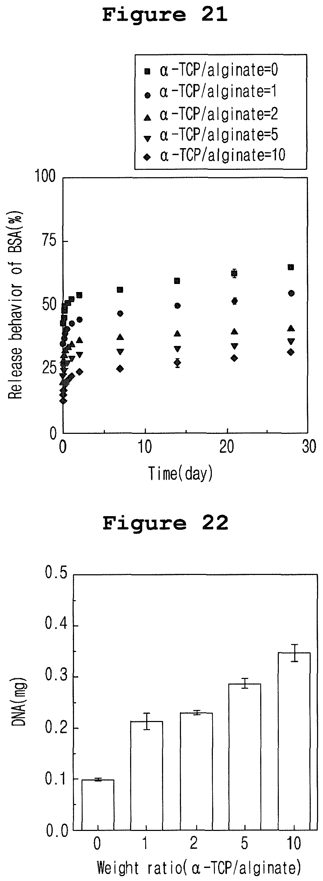

To confirm the feasibility of delivering growth factors, a protein form for inducing tissue differentiation, BSA was added to the solutions of Comparative Example 1 and Examples 2 to 5, photocrosslinked by UV irradiation, and then immersed in a PBS (phosphate buffer saline) curing liquid for about 24 hours at 37°C to prepare an organic-inorganic complex hydrogel, and the initial drug loading efficiency and release behavior for one month were confirmed. The results are shown in Figures 20 and 21.

Figure 20 is a graph showing the initial drug loading efficiency, confirming that the amount of protein remaining after the initial curing for 24 hours was increased as the ratio of the ceramic material in the organic-inorganic complex hydrogel was increased.

In addition, Figure 21 is a graph showing the release behavior for one month, confirming that protein release could be induced from the organic-inorganic complex hydrogel for more than one month.

### <Experimental Example 9> Osteogenic differentiation induction characteristics

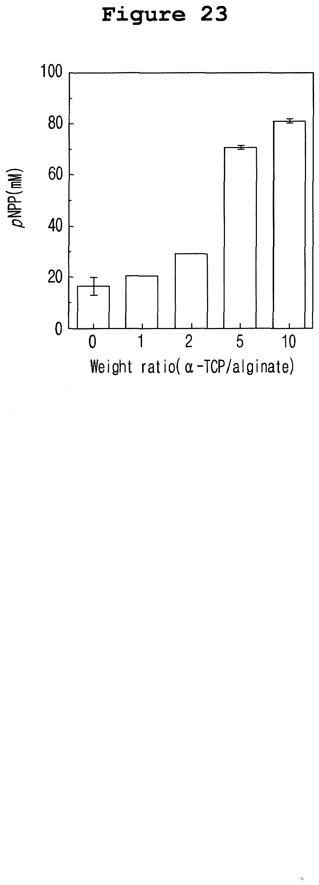

To confirm the possibility of inducing osteogenic differentiation as a functional bone substitute, MG-63 cells were cultured for 2 weeks on the surface of the organic-inorganic complex hydrogel prepared by the method of Example 7 using the solutions of Examples 2 to 5, and DNA and ALP activity of the cells on the surface were confirmed. The results are shown in Figures 22 and 23.

As shown in Figures 22 and 23, it was confirmed that the higher the ceramic ratio, the higher the activity of ALP, an osteogenic differentiation factor, and in particular, when the weight of α-TCP compared to alginate was 5 times or more, it was confirmed that osteogenic differentiation was induced very rapidly.

### <Experimental Example 10> Evaluation of curing characteristics after injection into living tissue

In order to confirm the characteristics of curing by UV and a curing liquid after injection into the living tissue, the following experiment was performed.

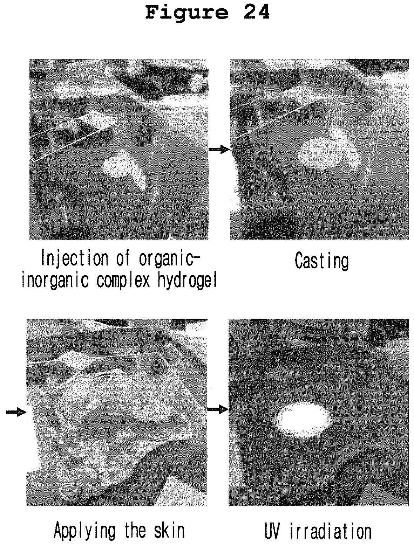

Figure 24 is a photograph showing the experimental process for confirming curing characteristics by UV after injection into the living tissue.

As shown in Figure 24, each of the composition for preparing an organic-inorganic complex hydrogel of Examples 1 to 5 and the composition of Comparative Example 1 was dropped on a glass substrate, the skin of an experimental rat was applied so that the composition was placed under the skin of the rat, and the skin of the rat was irradiated with UV to determine whether the UV passed through the skin of the rat to produce a hydrogel from the composition.

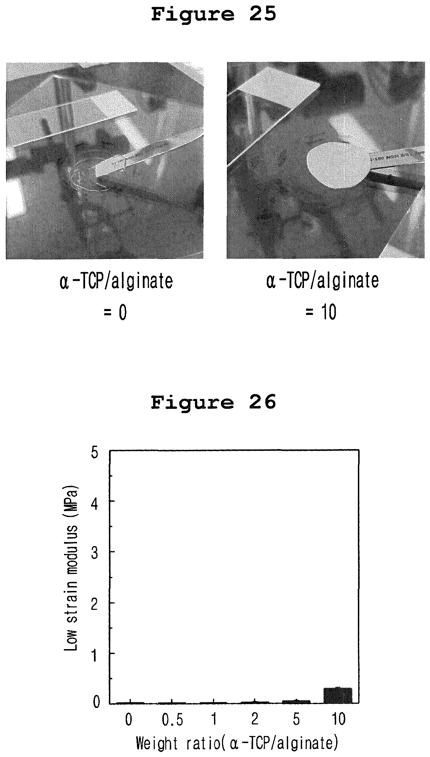

As a result of the experiment, it was confirmed that UV passed through the skin of the rat and formed a hydrogel from the composition for preparing an organic-inorganic complex hydrogel of Examples 1 to 5 and the composition of Comparative Example 1. The results of Example 5 and Comparative Example 1 are shown in Figure 25.

In addition, after immersing the hydrogels formed from the composition for preparing an organic-inorganic complex hydrogel of Examples 1 to 5 and the composition of Comparative Example 1 in saline for about 24 hours, a compressive strength measurement experiment was performed in the same manner as in Experimental Example 2, and the low modulus values of the stress-strain curve obtained therefrom were compared and the results are shown in Figure 26.

As shown in Figure 26, as a result of the compressive strength test, the hydrogels formed from the compositions of Examples 1 to 5 (α-TCP/alginate weight ratio=0.5~10) exhibited higher compressive strength than that of the hydrogel formed from the composition of Comparative Example 1 (α-TCP/alginate weight ratio=0) without α-TCP. In particular, when the weight of α-TCP compared to the weight of alginate was 5 to 10 times, the compressive strength was higher, and when it was 10 times, the compressive strength was significantly higher.

From the above results, it was confirmed that the composition for preparing an organic-inorganic complex hydrogel according to one embodiment can be injected into an animal and then cured into a hydrogel by UV irradiation, and then the strength can be improved by a curing liquid such as saline.

## Claims

1. A composition for preparing an organic-inorganic complex hydrogel comprising a biocompatible polymer having a photocrosslinkable functional group; and a calcium phosphate-based ceramic powder.

2. The composition for preparing an organic-inorganic complex hydrogel according to claim 1, wherein the weight ratio of the biocompatible polymer and the calcium phosphate-based ceramic powder is1:1 or more and less than 1:20.

3. The composition for preparing an organic-inorganic complex hydrogel according to claim 1, wherein the biocompatible polymer is at least one selected from the group consisting of alginate, hydroxypropylmethylcellulose (HPMC), hydroxyethylcellulose (HEC), methyl cellulose, carboxymethylcellulose (CMC), gelatin, collagen, fibrinogen, chitosan, agar, matrigel, starch, pectin, polyvinyl alcohol, polyurethane, poly(ethylene glycol), poly(propylene glycol), hyaluronan and poly(vinylpyrrolidone).

4. The composition for preparing an organic-inorganic complex hydrogel according to claim 1, wherein the calcium phosphate-based ceramic powder is at least one selected from the group consisting of α-TCP (a-tricalcium phosphate), β-TCP (β-tricalcium phosphate), hydroxyapatite, DCPD (dicalcium phosphate dihydrate), MCPM (monocalcium phosphate monohydrate), DCPA (dicalcium phosphate anhydrous) and BCP (biphasic calcium phosphate).

5. The composition for preparing an organic-inorganic complex hydrogel according to claim 1, wherein the calcium phosphate-based ceramic powder is α-TCP (a-tricalcium phosphate).

6. The composition for preparing an organic-inorganic complex hydrogel according to claim 1, wherein the composition further includes at least one of a functional member and a cell.

7. A kit for preparing an organic-inorganic complex hydrogel comprising a composition for preparing an organic-inorganic complex hydrogel comprising a biocompatible polymer having a photocrosslinkable functional group and a calcium phosphate-based ceramic powder; and a curing liquid.

8. The kit for preparing an organic-inorganic complex hydrogel according to claim 7, wherein the weight ratio of the biocompatible polymer and the calcium phosphate-based ceramic powder is 1 : 1 or more and less than 1:20.

9. The kit for preparing an organic-inorganic complex hydrogel according to claim 7, wherein the curing liquid is at least one selected from the group consisting of saline, PBS (phosphate buffer saline), MCPM (monocalcium phosphate monohydrate), DSP (disodium phosphate dehydrate), MSP (monosodium phosphate dehydrate), a-MEM (minimum essential medium) and HBSS (Hank's balanced salt solution).

10. The kit for preparing an organic-inorganic complex hydrogel according to claim 7, wherein the curing liquid further includes calcium ions (Ca²⁺).

11. The kit for preparing an organic-inorganic complex hydrogel according to claim 7, wherein the kit further includes at least one of a functional member and a cell.

12. An organic-inorganic complex hydrogel comprising a biocompatible polymer having a photocrosslinkable functional group; and a calcium phosphate-based ceramic bonded to the biocompatible polymer.

13. The organic-inorganic complex hydrogel according to claim 12, wherein the calcium phosphate-based ceramic is CDHA (Ca-deficient hydroxyapatite).

14. A biomaterial comprising the organic-inorganic complex hydrogel of claim 12.
